# EUROPEAN PATENT APPLICATION

(11) **EP 2 650 363 A1**
(43) Date of publication of application: **16.10.2013**
(21) Application number: 11846384.3
(22) Date of filing: 18.11.2011
(51) Int. Cl.: C12N 9/02, C12N 9/04, C12H 1/15

(54) **METHOD FOR THE PURIFICATION AND STABILISATION OF ENZYME GLUCONATE DEHYDROGENASE (GADH, EC 1.1.99.3), ENZYME GLUCONATE DEHYDROGENASE (GADH, EC 1.1.99.3) AND THE USE OF ENZYME GLUCONATE DEHYDROGENASE (GADH, EC 1.1.99.3)**

(30) Priority: 10.12.2010 ES 201031819
(71) Applicant: Biolan Microbiosensores, S.L., 48160 Derio (Bizkaia) (ES)
(72) Inventor: ALONSO RODRIGUEZ, Pablo, E-48160 Derio (Bizkaia) (ES); QUIROS FERNANDEZ, Luis Manuel, E-48160 Derio (Bizkaia) (ES); CASTAÑON DE LA TORRE, Sonia, E-48160 Derio (Bizkaia) (ES); CRESPO SUSPERREGUI, Ainara, E-48160 Derio (Bizkaia) (ES); MAZA DEL RIO, Sonia, E-48160 Derio (Bizkaia) (ES)
(74) Representative: Urizar Barandiaran, Miguel Angel
(86) International application number: PCT/ES2011/070794
(87) International publication number: WO 2012/076738

(57) **Abstract**

The invention relates to a method for the purification and stabilisation of enzyme gluconate dehydrogenase (GADH, EC 1.1.99.3), optionally recombinant, from multiple microorganisms, for example: Pseudomonas aeruginosa, Pseudomonas fluorescens, Gluconobacter oxydans, Gluconobacter industrius, Serratia marcescens, Klebsiella pneumoniae and Escherichia coli, and to the use thereof as a biological recognition element in biosensors for detecting gluconic acid in samples of interest. The invention also relates to a biocatalytic biosensor with free electrochemical transduction of interferences owing to the high selectivity of the enzyme obtained using the aforementioned method and stabilised with optimised chemical agents.

## Description

### STATE OF THE ART

In comparison to conventional analytical methods (HPLC with several detectors, atomic absorption spectrophotometry, polarography...etc.) bioanalytical methods have become of great interest due to the high selectivity of the elements of biological recognition. The most relevant example of this increase in interest is the revolutionary invention of biosensors from 1962, thanks to the work of Clark and Lyon [L.C. Clark and C. Lyons, Ann. NY. Acad. Sci 120 (1962) 29]. These are compact devices that are based on the close integration of elements of biological recognition in a system of transduction of the physical signal [D.R Thévenot et al Pure. Appl. Chem 71(1999)2333]. The development of these devices passes generically through three lines of research: the choice of the suitable system of transduction of the physical signal, the immobilization and/or the integration of this element in the sensor surface, the correlation of the signal generated with the presence of the target analyte. These compact devices are affected by the presence of interfering elements present in the matrix, which can be palliated with chemometric developments or applying materials compatible with the ERB to the biosensor.

In current bibliographic work many inventions can be found centered on the foregoing approximation, the application of protective layers that serve to both protect the ERBs from the possible inhibitors and the system of transduction of the signal from the interfering elements. In fact, biocompatible polymers have been used abundantly and with great success, such as cellulose acetate [H. Gunasingham et al, Biosensors 4 (1989)349; X. Ren et al Colloids Surf B Biointerfaces 72 (2009)188; R. Vaidya and E. Wilkins, Electroanalysis 6 (1994) 617; L.N. Wu et al Electrochim. Acta 51 (2006) 1208], chitosan [J. Lin et al Sensors Actuators B: Chem 137 (2009) 768; S. Hikima et al, Fereseniu's. J. Anal. Chem 345 (1993) 607; H. Yu et al, Anal. Biochem 331 (2004) 98; G. Wang et al Biosens. Bioelectron 18 (2003) 335; X. Kang et al Biosens. Bioelectron 25 (2009) 901; J. Chem, Electroanalisis 18 (2006) 670] and Nafion [M. ElKaoutit and Col WO/2009/022035; M. ElKaoutit and Col WO/2009/034200; J. Wang et al, J. Am. Chem. Soc 125(2003) 2408; S. H. Lim, Biosens. Bioelectron 20 (2005) 2341; Y-C. Tsai et al Langmuir 21 (2005) 3653; A. A Karyakin et al, Anal. Chem 72 (2000) 1720; M. ElKaoutit et al J. Agric. Food. Chem 55 (2007)8011; M. ElKaoutit et al, Talanta 75 (2008) 1348; M. ElKaoutit et al, Biosens. Bioelectron 22 (2007) 2958].

This invention has been centered on a totally novel approximation to prevent problems of inhibition of the enzyme Gluconate Dehydrogenase (GADH, 1.1.99.3), to protect it and give it certain durability at room temperature in a typically complex matrix such as wine or grape juice. It is an important fact that the sensitivity to certain inhibitors and interfering elements can depend on the organism from which this element of biological recognition is extracted, on its purification process and even on the stabilizers necessary to maintain a certain structural conformation of the protein. The purification of the enzyme GADH (1.1.99.3) has been obtained, by very novel methods, from several microorganisms, and its stabilization in a specific solution. As an application, biosensors have been manufactured with simple physical retention on a conductive base of a liquid aliquot of this enzyme with a dialysis membrane together with the chemical mediator; without application of any matrix of immobilization or a protector. The resulting device has shown satisfactory selectivity and stability in gluconic acid as a substrate in typically complex matrices such as wine (very rich in en tannins and sulphites) and/or grape juice (with a high level of glucose and ascorbic acid, for example).

This invention advocates a process of purification and stabilization of the enzyme Gluconate Dehydrogenase (GADH, EC 1.1.99.3) either recombinant or not, in which the purification of GADH is carried out from cells of *Serratia* marcescens, *Kleibsella pneumoniae, Pseudomonas aeruginosa, Pseudomonas fluorescens, Gluconobacter oxydans, Gluconobacter industrius* or Eschericia coli or mixture of them, and in which the cellular breakage is produced by sonication or another type of physical breakage, then obtaining their membranes, in which:
a) the membranes are resuspended by extrusion and
b) The solubilization of the enzyme GADH of the membrane is realized by the addition of detergents such as n-Octyl-β-D-thioglucoside, Zwittergent 3-12, Twenn 80, Brij 58 or Triton X-100 in v/v percentages between 0.1% and 3 %, suspension is ultracentrifugated and the supernatant is collected,
c) The supernatant is subjected to a chromatography of ion exchange at a pH between 4 and 8.5.

It is also characterized in that to the purified enzyme GADH the following are added:
- gluconic acid at a concentration between 5 and 20 mM;
- a v/v concentration of glycerol between 10 and 50%;
- detergents such as n-Octyl-β-D-thioglucoside, Zwittergent 3-12, Twenn 80, Brij 58 or Triton X-100 at a v/v concentration between 0.05 and 2%;
- a divalent cation that can be MgCl2, CaCl2 or BaCl2 at a concentration between 1 and 10 mM; and
- one or several of the following carbohydrates in a v/v concentration between 1 and 20%; trehalose, malitol, mannitol, sorbitol, dextran and Ficoll™.

The Enzyme Gluconate Dehydrogenase (GADH) obtained according to this process is also an object of the invention.

Likewise, the use of the enzyme is the object of the invention, either obtained or not, according to the Process described above, as an element of biological recognition for the development of devices for measurement of the enzymatic substrate gluconic acid.

The use of the enzyme, either obtained or not, according to the above mentioned process, to measure the values of gluconic acid in food samples, such as grape juices and wines, is also an object of the invention.

### Explanation of a preferential form of embodiment

For a better understanding of this invention, the following examples are explained, described in detail, the nature of which must be understood as not limiting the scope of this invention.

### Example 1. Purification of gluconate 2-dehydrogenase (GADH, 1.1.99.3) from Pseudomonas aeruginosa

The bacterial strain used for this invention, *Pseudomonas aeruginosa* CECT 108, can be cultivated in any medium that induces the pentose phosphate cycle.

In the production step, two 2-litre Erlenmeyer flasks were used, each one containing 600 ml of medium, which had been inoculated with 10% volume of culture grown in the same medium until obtaining a OD₆₀₀=2,5. The cultures were incubated at 28°C until their late exponential phase (approximately 12 hours).

The cultures were centrifugated for 45 minutes at 9000 rpm, the supernatant was eliminated and the precipitate was collected. The cell mass was kept frozen at - 80°C until the moment of its rupture.

To begin the rupture of the cells, they were thawed and were resuspended in 5 times their volume in an acetate buffer 50 mM and a pH=4.5 rupture. 5 mg/ml of lyzozyme and protease inhibitor were added and were kept in agitation at room temperature for 30 minutes. At that moment, 0.5 ml of DNAsa II 1 mg/ml were added in NaCl 0,15 M and the cells were kept in agitation in the described buffer at 4°C until the following day.

Later the cells were subjected to 6 pulses of sonication, with 50% amplitude. They were centrifugated, to separate the unbroken cells and the cell walls (precipitate) of the supernatant, which contained the cytoplasmatic proteins and the cell membranes in suspension. Collection of the supernatant of larger density, which remained on the precipitate, was avoided due to the difficulty involved in the separation of the cytoplasmatic proteins from the membrane proteins.

The supernatant was ultracentrifugated at 20000 rpm, after which the supernatant was discarded and the precipitate was again resuspended in acetate buffer 50 mM pH=4.5 to eliminate the greatest possible quantity of cytoplasmatic proteins retained in the precipitate membranes. It was ultracentrifugated at 20000 rpm and the supernatant was again discarded.

The membranes were resuspended this time in acetate buffer 20 mM pH=4.5 approximately 2 ml. To homogenize the suspension they were subjected to an ultrasound bath, preventing the heating of the sample through the insertion of the glass tube that contained it in flake ice.

At that moment, protein was quantified by Bradford reagent on spectrophotometer at 595 nm wave length and enzymatic activity, also on spectrophotometer, at 600 nm for 5 minutes. The reagents used for the enzymatic test were 700 µl of phosphate buffer 135 mM pH=4.5, 100 µl of gluconate 165 mM in the same phosphate buffer, 100 µl of DCPIP 2.2 mM and 100 µl of PMS 13 mM.

The volume of resuspension buffer was increased until obtaining a protein concentration of 10 mg/ml. Triton X-100 detergent at 0.5% was added to solubilize the membrane proteins and it was kept in agitation at 4°C during the night.

The following day, the suspension was ultracentrifugated at 35000 rpm. The precipitate was discarded, the supernatant was collected and the enzymatic activity was again measured.

The supernatant was subjected to chromatography of ion exchange on a FPLC unit, using as buffer A: acetate 20 mM pH=4.5 and Triton X100 at 0.1% and, as buffer B, the same, adding KCl 1M. The sample was eluted with a gradient of 0 to 50%, with a flow of 1 ml/min. Enzymatic test and quantification of protein of the eluted fractions were performed, to make a selection of those fractions with greater specific activity.

The enzyme was concentrated by ultrafiltration in a membrane of 50000 MWCO diameter cut-off until obtaining an approximate concentration of 0.2 UE/µl.

For its conservation, as a solvophobic agent, 15% glycerol was added and detergent, 1% Triton X-100, a cryoprotectant agent, 1% Trehalose and stabilizers with a base of divalent cations and/or natural substrates of the enzyme.

### Example 2. Purification of gluconate 2-dehydrogenase (GADH, 1.1.99.3) from Gluconobacter Oxydans.

The bacterial strain used for this invention, Gluconobacter oxydans CECT 360, can be cultivated in any medium that induces the pentose phosphate cycle.

In the production step, six 2-litre Erlenmeyer flasks were used, each one containing 600 ml of medium, which had been inoculated with 10% volume of culture grown in the same medium until obtaining a OD₆₀₀=2. The cultures were incubated at 30°C until their late exponential phase (approximately 48 hours).

The cultures were centrifugated for 45 minutes at 9000 rpm, the supernatant was eliminated and the precipitate was collected. The cell mass was kept frozen at - 80 °C until the moment of its rupture.

To begin the rupture of the cells, they were thawed and were resuspended in 5 times their volume in phosphate buffer 100 mM pH=6. 5 mg/ml of lyzozyme and protease inhibitor were added and were kept in agitation at room temperature for 30 minutes. At that moment, 0.5 ml of DNAsa II 1 mg/ml were added in NaCl 0,15 M and the cells were kept in agitation in the described buffer at 4°C until the following day.

Later, the cells were thawed and were subjected to two passages through French press at 1000 Kg/cm². They were centrifugated, to separate the unbroken cells and the cell walls (precipitate) of the supernatant, which contained the cytoplasmatic proteins and the cell membranes in suspension. Collection of the supernatant of larger density, which remained on the precipitate, was avoided due to the difficulty involved in the separation of the cytoplasmatic proteins from the membrane proteins.

The supernatant was ultracentrifugated at 20000 rpm, after which the supernatant was discarded and the precipitate was again resuspended in phosphate buffer 50 mM pH=6 to eliminate the greatest possible quantity of cytoplasmatic proteins retained in the precipitate membranes. It was ultracentrifugated at 20000 rpm and the supernatant was again discarded.

The membranes were resuspended, this time in phosphate buffer 20 mM pH=7, approximately 6 ml. To homogenize the suspension they were subjected to an ultrasound bath, preventing the heating of the sample through the insertion of the glass tube that contained it in flake ice.

At that moment, protein was quantified by Bradford reagent on spectrophotometer at 595 nm wave length and enzymatic activity, also on spectrophotometer, at 600 nm for 5 minutes. The reagents used for the enzymatic test were 700 µl of phosphate buffer 135 mM pH=5, 100 µl of gluconate 165 mM in the same phosphate buffer, 100 µl of DCPIP 2.2 mM and 100 µl of PMS 13 mM.

The buffer volume of resuspension was increased until obtaining a protein concentration of 10 mg/ml. Detergent Twenn 80 at 0.5% was added to solubilize the membrane proteins and it was kept in agitation at 4 °C during the night.

The following day, the suspension was ultracentrifugated at 35000 rpm. The precipitate was discarded, the supernatant was collected and the enzymatic activity was again measured.

The supernatant was subjected to chromatography of ion exchange on a FPLC unit, using as buffer A: phosphate 20 mM pH=7 and Twenn 80 at 0.1 % and, as buffer B, the same, adding KCI 1 M. The sample was eluted with a gradient of 0 to 50%, with a flow of 1 ml/min. Enzymatic test and quantification of protein of the eluted fractions were performed, to make a selection of those fractions with greater specific activity.

The enzyme was concentrated by ultrafiltration in a membrane of 50000 MWCO diameter cut-off until obtaining an approximate concentration of 0.2 UE/µl.

For its conservation, as a solvophobic agent 15% of glycerol was added and detergent of 1% Twenn 80, a cryoprotector agent of 3% Dextran 40 and stabilizers with a base of divalent cations and/or natural substrates of the enzyme.

### Example 3. Purification of gluconate 2-dehydrogenase (GADH, 1.1.99.3) from Serratia marcescens.

The bacterial strain used for this invention, *Serratia marcescens* IFO 3054, was cultivated in a medium that contained 0.1% polypeptone, 0.1% yeast extract, 0.1 % NaCl, 0.3% KH₂PO₄, 0.04% Na₂SO₄ and 0.04 MgSO₄ x 7H₂O.

In the production step, two 2-litre Erlenmeyer flasks were used, each one containing 600 ml of medium, which had been inoculated with 10% volume of culture grown in the same medium until obtaining OD₆₀₀=3. The cultures were incubated at 26 °C until their late exponential phase (approximately 48 hours).

The cultures were centrifugated for 45 minutes at 9000 rpm, the supernatant was eliminated and the precipitate was collected. The cell mass was kept frozen at - 80 °C until the moment of its rupture.

To begin the rupture of the cells, they were thawed and were resuspended in 5 times their volume in phosphate buffer 100 mM pH=6. 5 mg/ml of lyzozyme and protease inhibitor were added and were kept in agitation at room temperature for 30 minutes. At that moment, 0.5 ml of DNAsa II 1 mg/ml were added in NaCl 0,15 M and the cells were kept in agitation in the described buffer at 4°C until the following day.

Later the cells were thawed and were subjected to two passages through French press at 1000 Kg/cm². They were centrifugated, to separate the unbroken cells and the cell walls (precipitate) of the supernatant, which contained the cytoplasmatic proteins and the cell membranes in suspension. Collection of the supernatant of larger density, which remained on the precipitate, was avoided due to the difficulty involved in the separation of the cytoplasmatic proteins from the membrane proteins.

The supernatant was ultracentrifugated at 20000 rpm, after which the supernatant was discarded and the precipitate was again resuspended in phosphate buffer 50 mM pH=6 to eliminate the greatest quantity possible of cytoplasmatic proteins retained in the precipitate membranes. It was ultracentrifugated at 20000 rpm and the supernatant was again discarded.

The membranes were resuspended this time in acetate buffer 20 mM pH=5, approximately 2 ml. To homogenize the suspension the membranes were subjected to extrusion through a syringe.

At that moment, protein was quantified by Bradford reagent on spectrophotometer at 595 nm wave length and enzymatic activity, also in spectrophotometer, at 600 nm for 5 minutes. The reagents used for the enzymatic test were 700 µl of phosphate buffer 135 mM pH=4.5, 100 µl of gluconate 165 mM in the same phosphate buffer, 100 µl of DCPIP 2.2 mM and 100 µl of PMS 13 mM.

The buffer volume of resuspension was increased until obtaining a protein concentration of 15 mg/ml. Detergent n-Octyl-β-D-thioglucoside at 2% was added to solubilize the membrane proteins and it was kept in agitation at 4°C during the night.

The following day, the ultrasuspension was ultracentrifugated at 35000 rpm. The precipitate was discarded, the supernatant was collected and the enzymatic activity was again measured.

The supernatant was subjected to chromatography of ion exchange in a FPLC unit, using as buffer A: phosphate 20 mM pH=7 and n-Octyl-β-D-thioglucoside at 0.1 % and, as buffer B, the same, adding KCI 1 M. The sample was eluted with a gradient of 0 to 50%, with a flow of 1 ml/min. Enzymatic test and quantification of protein of the eluted fractions were performed, to make a selection of those fractions with greater specific activity.

Finally, the enzyme was concentrated by ultrafiltration in membrane of 50000 MWCO diameter cut-off until obtaining an approximate concentration of 0.2 UE/µl.

For its conservation, as a solvophobic agent, 15% glycerol was added and detergent, 1% of n-Octyl-β-D-thioglucoside, a cryoprotector agent 10% of Ficoll and stabilizers with a base of divalent cations and/or natural substrates of the enzyme.

### Abbreviations:

- DNAsa:: deoxyribonuclease
- OD₆₀₀:: optical density at 600 nm.
- r.p.m.:: revolutions per minute
- DCPIP:: dichlorophenolindofenol
- PMS:: phenazine methosulfate
- MWCO:: cut-off molecular weight
- FPLC:: Liquid chromatography for proteins

### Application

The obtained in this way has been tested in the development of a second-generation electro-bio-electrocatalytic biosensor in wines and grape juices in amperometric mode and the results have been satisfactory.

## Claims

1. Process of purification and stabilization of the enzyme Gluconate Dehydrogenase (GADH, EC 1.1.99.3) either recombinant or not, in which the purification of GADH is carried out from cells of *Serratia marcescens, Kleibsella pneumoniae, Pseudomonas aeruginosa, Pseudomonas fluorescens, Gluconobacter oxydans, Gluconobacter industrius* or Eschericia coli or a mixture of them, and in which the cellular breakage is produced by sonication of another type of physical breakage, then obtaining their membranes, **characterized in that**:
a) the membranes are resuspended by extrusion and
b) the solubilization of the enzyme GADH of the membrane is realized by the addition of detergents such as n-Octyl-β-D-thioglucoside, Zwittergent 3-12, Twenn 80, Brij 58 or Triton X-100 in v/v percentages between 0.1 % and 3 %, the suspension is ultracentrifugated and the supernatant was collected,
c) the supernatant is subjected to a chromatography of ion exchange at a pH between 4 and 8.5.

2. Process of purification and stabilization of the enzyme Gluconate Dehydrogenase (GADH, EC 1.1.99.3) either recombinant or not, according to claim 1, **characterized in that** to the purified enzyme GADH gluconic acid is added at a concentration between 5 and 20 mM.

3. Process of purification and stabilization of the enzyme Gluconate Dehydrogenase (GADH, EC 1.1.99.3) either recombinant or not, according to claim 2, **characterized in that** to the purified enzyme GADH is added a v/v concentration of glycerol between 10 and 50%.

4. Process of purification and stabilization of the enzyme Gluconate Dehydrogenase (GADH, EC 1.1.99.3) either recombinant or not,, according to any of the claims 1 to 3, **characterized in that** to the purified enzyme GADH detergents are added such as n-Octyl-β-D-thioglucoside, Zwittergent 3-12, Twenn 80, Brij 58 or Triton X-100 at a v/v concentration between 0.05 and 2%.

5. Process of purification and stabilization of the enzyme Gluconate Dehydrogenase (GADH, EC 1.1.99.3) either recombinant or not, according to any of the claims 1 to 4, **characterized in that** to the purified enzyme GADH a divalent cation is added that can be MgCl2, CaCl2 or BaCl2 at a concentration between 1 and 10 mM

6. Process of purification and stabilization of the enzyme Gluconate Dehydrogenase (GADH, EC 1.1.99.3) either recombinant or not, according to any of the claims 1 to 5, **characterized in that** to the purified enzyme GADH is added one or several of the following carbohydrates in a v/v concentration between 1 and 20%; trehalose, malitol, mannitol, sorbitol, dextran and Ficoll™.

7. Enzyme Gluconate Dehydrogenase (GADH, EC 1.1.99.3) either recombinant or not, obtained according to the process of the foregoing claims.

8. Use of the enzyme Gluconate Dehydrogenase (GADH, EC 1.1.99.3) either recombinant or not, to measure the values of gluconic acid in food samples.
